# EUROPEAN PATENT APPLICATION

(11) **EP 4 783 182 A1**
(43) Date of publication of application: **29.07.2026**
(21) Application number: 25154052.2
(22) Date of filing: 27.01.2025
(51) Int. Cl.: G16H 20/40, G16H 40/63, G16H 50/50, A61N 5/06

(54) **SYSTEM AND METHOD FOR PREDICTING SETTINGS FOR TREATMENT OF TISSUE AND TISSUE RESPONSE**

(71) Applicant: Koninklijke Philips N.V., 5656 AE Eindhoven (NL)
(72) Inventor: VAN DEN DUNGEN, Wilhelmus Andreas Marinus Arnoldus Maria, Eindhoven (NL); STOFFELS, Monique, Eindhoven (NL); PAULUSSEN, Elvira Johanna Maria, 5656AG Eindhoven (NL); KOOIJMAN, Gerben, Eindhoven (NL)
(74) Representative: Philips Intellectual Property & Standards

(57) **Abstract**

A system for predicting tissue treatment settings and response of tissue is described. When executed by the processor, instructions cause the processor to: (a) receive measured nitric oxide (NO) tissue data from a personal healthcare device; (b) apply a trained computational model to predict an NO response, and based on the predicted NO response, predict tissue treatment settings; (c) compare the predicted treatment settings to a safety limit; (d) when the comparison of the predicted treatment settings is equal to or below the safety limit, update the predicted treatment settings and carrying out the tissue treatment at the predicted treatment settings; and (e) when the comparison of the prediction is greater than the safety limit, adjust the tissue treatment settings, update weights of the trained computational model, and repeat (c) through (e). A method and a tangible, non-transitory computer readable medium to make the predictions are also disclosed.

## Description

### BACKGROUND

Light therapy is being investigated to treat oral tissue. For example, certain wavelengths of light have been found to inhibit the growth of oral biofilms, thereby reducing dental plaque in a subject. Additionally, certain wavelengths of light have been found to reduce the inflammation response of human gingival cells. Accordingly, application of such light can reduce gingival inflammation (periodontitis).

While light treatment therapy shows promise for reducing inflammation of a variety of tissues, using certain known devices, the time required to gather data related to the inflammation can be quite long. Moreover, after data are gathered, it may be difficult to determine the correct treatment, and particularly, the correct settings of the device used to provide the treatment. As a result, known methods of diagnosing and effectively treating inflamed tissue are rather inefficient.

What is needed, therefore, is a method and system adapted to treat tissue that overcomes at least the known shortcomings of the known methods described above.

### SUMMARY

In accordance with a representative embodiment, method of predicting tissue treatment settings and (NO) response of tissue is disclosed. The method comprises: (a) measuring nitric oxide (NO) tissue data from a personal healthcare device comprising fluorescent dots (Fdots); (b) applying a trained computational model to the NO tissue data to predict an NO response, and based on the predicted NO response, predicting tissue treatment settings; (c) comparing the predicted treatment settings to a safety limit; (d) when the comparing of the predicted treatment settings is equal to or below the safety limit, updating the predicted treatment settings and carrying out tissue treatment at the predicted treatment settings; and (e) when the comparing of the prediction is greater than the safety limit, adjusting the tissue treatment settings, updating weights of the trained computational model, and repeating (c) through (e).

In accordance with another representative embodiment, a system for predicting tissue treatment settings and response of tissue is disclosed. The system comprises: a personal healthcare device comprising fluorescent dots (Fdots) adapted to detect nitric oxide (NO); a memory adapted to store a trained computational model comprising instructions; and a processor, wherein the instructions, when executed by the processor, cause the processor to:
(a) receive measured nitric oxide (NO) tissue data from a personal healthcare device; (b) apply a trained computational model to predict an NO response, and based on the predicted NO response, predict tissue treatment settings; (c) compare the predicted treatment settings to a safety limit; (d) when the comparison of the predicted treatment settings is equal to or below the safety limit, update the predicted treatment settings and carrying out the tissue treatment at the predicted treatment settings; and (e) when the comparison of the prediction is greater than the safety limit, adjust the tissue treatment settings, update weights of the trained computational model, and repeat (c) through (e).

In accordance with another representative embodiment, a tangible, non-transitory computer readable medium stores instructions, which when executed by a processor, cause the processor to: (a) receive measured nitric oxide (NO) tissue data from a personal healthcare device; (b) apply a trained computational model to the NO tissue data to predict an NO response, and based on the predicted NO response, predict tissue treatment settings; (c) compare the predicted treatment settings to a safety limit; (d) when the comparison of the predicted treatment settings is equal to or below the safety limit, update the predicted treatment settings and carrying out the tissue treatment at the predicted treatment settings; and (e) when the comparison of the prediction is greater than the safety limit, adjust the tissue treatment settings, update weights of the trained computational model, and repeat (c) through (e).

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 is a conceptual view showing sources of inflammation, resulting in production of NO, and resulting adverse effects.
Fig. 2A is a perspective view of a mouthpiece comprising a material comprising fluorescing dots (Fdots) in accordance with a representative embodiment.
Fig. 2B is a graph showing Fdot size versus emission wavelength.
Fig. 3 is a graph showing onset of NO production in the presence of blue light having a wavelength of 450 nm versus time.
Fig. 4 is a simplified block diagram of a system for evaluating and/or treating oral tissue using an inventive mouthpiece, in accordance with a representative embodiment.
Fig. 5A is a conceptual view of a system for evaluating and/or treating oral tissue using an inventive device in accordance with a representative embodiment.
Fig. 5B is a flow chart of method for evaluating and/or treating oral tissue using an inventive device in accordance with a representative embodiment.
Fig. 6A is a conceptual view of a system for evaluating and/or treating oral tissue using an inventive device in accordance with a representative embodiment.
Fig. 6B is a flow chart of method for evaluating and/or treating oral tissue using an inventive device in accordance with a representative embodiment.
Fig. 7 is a conceptual diagram showing training and execution of a computational model for predicting tissue treatment settings and (NO) response of tissue in accordance with a representative embodiment.
Fig. 8 is a flow-diagram of baseline training of a computational model for predicting a health status of tissue, tissue treatment settings and (NO) response of tissue, execution of the computational model, and updating of the model, in accordance with a representative embodiment.
Fig. 9A is a flow-diagram of baseline training of the computational model for predicting a health status of tissue in accordance with a representative embodiment.
Fig. 9B is a flow-diagram of baseline training of the computational model for predicting a tissue treatment settings and (NO) response of tissue in accordance with a representative embodiment.
Fig. 10 is a flow-diagram showing execution and updated training of a deployed computational model including personalization and federated learning based on a plurality of measurements of an individual and treatments of the individual in accordance with a representative embodiment.

### DETAILED DESCRIPTION

In the following detailed description, for the purposes of explanation and not limitation, representative embodiments disclosing specific details are set forth in order to provide a thorough understanding of embodiments according to the present teachings. However, other embodiments consistent with the present disclosure that depart from specific details disclosed herein remain within the scope of the appended claims. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. Definitions and explanations for terms herein are in addition to the technical and scientific meanings of the terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

As used in the specification and appended claims, the singular forms of terms 'a', 'an' and 'the' are intended to include both singular and plural forms, unless the context clearly dictates otherwise. Additionally, the terms "comprise", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to", or "coupled to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected or disposed immediately adjacent to each other without any intermediate or intervening elements or components.

As will be appreciated by one of ordinary skill in the art, nitric oxide (NO) is a signaling molecule that plays a role in the pathogenesis of inflammation. NO gives an anti-inflammatory effect under normal physiological conditions. On the other hand, NO is considered as a pro-inflammatory mediator that induces inflammation due to over production in abnormal situations. NO is synthesized and released into the endothelial cells by the help of NOSs that convert arginine into citrulline producing NO in the process. Oxygen and Nicotinamide adenine dinucleotide phosphate (NADPH) are necessary cofactors in such conversion. NO is believed to induce vasodilatation in cardiovascular system and furthermore, it involves in immune responses by cytokine-activated macrophages, which release NO in high concentrations. In addition, NO is a potent neurotransmitter at the neuron synapses and contributes to the regulation of apoptosis. NO is involved in the pathogenesis of inflammatory disorders of the joint, gut and lungs. Therefore, NO inhibitors represent important therapeutic advance in the management of inflammatory disease. Generally, the present teachings are directed to methods, systems and ML models (sometimes referred to herein as algorithms) used not only to diagnose inflammation based on NO emissions, but also to predict the appropriate settings for the treatment and the NO response of the tissue to the treatment.

As described herein, a device for detecting the presence of RNS or ROS emanating from tissue is described. As will become clearer as the present description continues, many of the representative embodiments are directed to detection of NO. It is emphasized that this is merely illustrative, and other RNS and ROS components are contemplated for detection by the device of the various representative embodiments. Notably, variation of the presently described embodiments to more generally detect RNS or ROS component, including the selection of material for the Fdots may be needed.

In certain representative embodiments, the device is adapted to treat regions where inflammation exists in the tissue. As such, by the present teachings, the device is adapted to not only detect the presence of inflammation in tissue, but also is adapted to provide treatment to the inflamed area and/or detect the effect of the treatment. As described more fully herein, the devices of the various representative embodiments comprise Fdots including so-called polymer dots (Pdots) and Qdots. The materials selected for the Fdots is adapted to emit fluorescent light when light of a certain wavelength is incident on the Fdots. The light that causes the fluorescent emission from the Fdots is referred to herein as an excitation light having an excitation wavelength. The light emitted from the Fdots during fluorescence is referred to herein as emission light and has an emission wavelength. Notably, the excitation wavelength is selected to provide treatment to inflamed tissue. Moreover, in certain representative embodiments, Fdots are selected so that in the presence of RNS including NO, and ROS, the fluorescent emission terminates. These Fdots may be referred to herein as "turn-off' Fdots. In other representative embodiment, the Fdots are selected to fluoresce in the presence of RNS or ROS. These Fdots may be referred to herein as "turn-on" Fdots. As such, according to various representative embodiments, the devices of the various representative embodiments function as a detector of RNS and ROS, which, as described more fully below, is indicative of such inflammation.

In certain representative embodiments, this detection provides not only the location of inflammation, but also the time of the detection. In other representative embodiments the emission light is used not only to detect RNS or ROS, but also to treat the inflamed tissue. Moreover, based on the detection of inflamed tissue, the output of the light source used for treating the inflamed tissue may be altered to better treat the inflammation. In addition, and as described more fully below, the Fdots may be used to monitor the progress of treatment of a condition. Specifically, as the treated tissue heals, the inflammation is reduced, and the production of NO decreases. In a representative embodiment, as the presence of NO decreases, and the fluorescent emission caused by the excitation light source increases providing a measure of the progress of the treatment. Accordingly, and among other improvements to the field of medical diagnosis and treatment, the present teachings enable the detection and monitoring of inflammation in tissue including internal and external tissue, monitoring of the progress of a treatment, and adaptation of treatment of tissue based on the monitoring.

In certain representative embodiments, trained computational models (also referred to herein as trained ML models) comprise an ML algorithm (again, also referred to herein as an ML model) and are stored in memory as instructions for execution by a processor. The computational models (again also referred to herein as ML models) of various embodiments are designed and trained to predict the health of tissue. Computational models of various embodiments are also designed and trained to improve light therapy treatments for an individual by predicting treatment settings (e.g., light wavelength, light intensity and duration of treatment) for the tissue based on the health of the tissue. Moreover, the trained computational models of various embodiments predict the NO response of the tissue to the treatment based on the predicted settings. As described more fully below in accordance with a representative embodiment, an ML algorithm is trained for an individual (subject) based on a baseline ML model. During training or retraining, model weights of the ML model can be updated for the individual. Moreover, and as described more fully below, a federated learning sequence allows the weights of the model to be shared in a way that does not compromise patient identity to improve the baseline model for other individuals. Finally, while the application of the ML algorithms of certain representative embodiments is made to gums and oral healthcare, the present teachings contemplate application to other tissue that emit NO caused by inflammation and healing of various tissue, including gums and wounds to the skin. As such, while various representative embodiments are directed to the use of a mouthpiece to measure NO emissions of gum tissue, the personal healthcare devices described herein are contemplated for tissue health assessment for other types of tissue, and in addition to a mouthpiece, the personal healthcare devices may comprise an oral hygiene device (e.g., a toothbrush), or a bandage, or similar devices comprising the Fdots to carry out the measurements of NO emission.

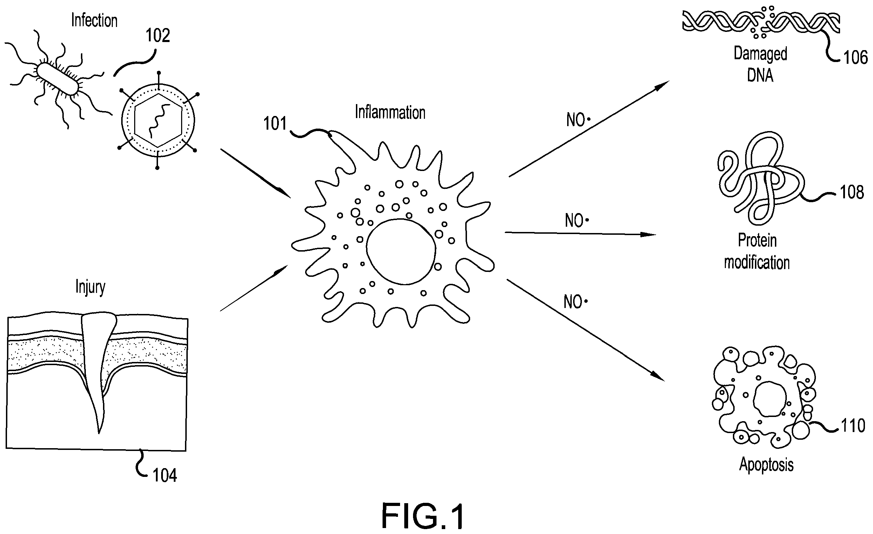

Fig. 1 is a conceptual view showing sources of inflammation 101 in a region of tissue, resulting in production of NO, and resulting adverse effects. Notably, in many of the representative embodiments described herein, the tissue that is inflamed is gingival tissue (e.g., gums), and the indicator of inflammation is NO. However, this is merely for ease of description. More generally, the tissue is not limited to the gums, and other tissue, such as skin, which is susceptible to inflammation, emits RNS or ROS when inflamed, and is beneficially treated with light are contemplated. In other applications, the progress of treatment of tissue can be monitored using Fdots of various representative embodiments. As described more fully below, as the treated area heals, inflammation is reduced, and the emission of NO from the inflamed tissue is accordingly reduced. When the Fdots turn off in the presence of NO, reduced NO caused by the healing of tissue results in an increase in fluorescent emission, and indicates the progress of healing by a particular treatment. Alternatively, when the Fdots turn on (fluoresce) in the presence of NO, and the NO is reduced as a result of the healing of tissue. In this case, a decrease in fluorescent emission is realized.

As shown in Fig. 1, the inflammation 101, which can result in different layers of tissue, may be caused by infection 102 or injury 104. The sources of injury are diverse, including but not limited to exposure to UVA radiation, UV-induced erythema, wound healing or other injury. As a result, the inflamed layers of skin can release NO. NO exists in vivo as a dissolved free radical gas comprising an unpaired electron on the Nitrogen that can inflict cellular damage under prolonged inflammation. For example, NO can cause DNA damage 106, protein modification 108 and apoptosis 110, as well as other possible deleterious effects on the body. As such, excessive NO levels during inflammation can lead to detrimental effects like protein nitration, DNA damage and programmed cell death.

The presence of NO in tissue signals inflammation, and as described more fully below, is detected not only to determine the location and/or time of inflammation, but also to determine the location and dose of treatment of the inflamed tissue useful given the condition of the tissue. Moreover, because light treatment of the tissue can result in the production of NO, the devices of the various representative embodiments may be used to monitor the light treatment in real-time. For example, the devices of the various representative embodiments can be used to determine when the light treatment is too intense and/or applied for too long. In this case, the light treatment can be reduced to avoid tissue damage.

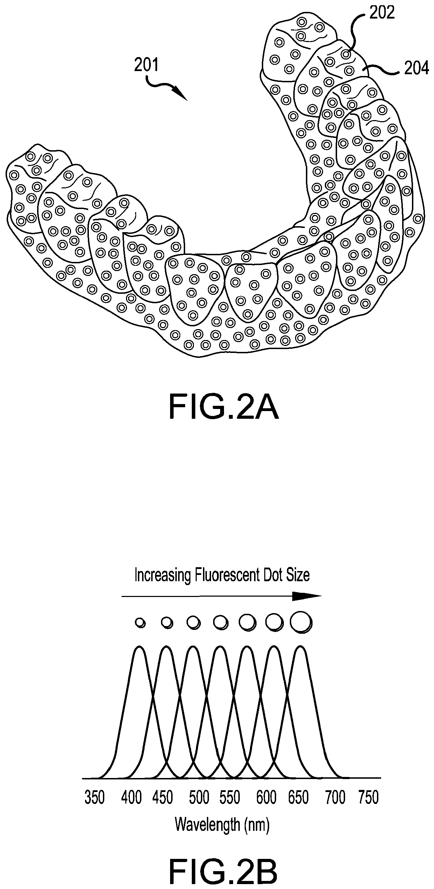

Fig. 2A is a perspective view of a mouthpiece 201 comprising a material 204 comprising fluorescing dots (Fdots) 202 in accordance with a representative embodiment. It is again emphasized that the use of Fdots in a mouthpiece for oral use is merely illustrative, and applications to other tissue are contemplated with appropriate safeguards as described more fully below.

Once detected, the areas of the tissue where inflammation exists can be treated with light of a particular wavelength useful in treating the underlying condition. Alternatively, or additionally, and again, as described more fully below, the Fdots may be used to monitor the progress of treatment of a condition. Specifically, as the treated tissue heals, the inflammation is reduced, and the production of NO decreases. As the presence of NO decreases, and the fluorescent emission caused by the excitation light source increases providing a measure of the progress of the treatment. Accordingly, in oral applications Fdot signal response to light-induced NO production provides a measure of light treatment status of the oral tissue (e.g., gum) that can be used to determine both the treatment and the progress of the treatment. In such way, severe inflammation (e.g., periodontitis) in the mouth can effectively be treated locally and the light treatment can be adapted and targeted to aim for better safety and effectiveness in a personalized way.

Another beneficial aspect of the various representative embodiments includes the improved ability to monitor the temperature of the tissue. Notably, in certain known medical measurements, the temperature is used to identify areas where tissue damage is imminent. These known methods provide only a course measure, with a threshold of 43 °C being the benchmark above which damage can occur. It is noted that one source of inflammation can be the applied radiation (light) used in the treatment of the tissue. Beneficially, the devices and systems of the various representative embodiments are adapted to provide a more accurate measure of the change in the temperature of the tissue undergoing treatment and more promptly. Specifically, by monitoring the rise in the temperature, the applied light dose could be altered before the temperature reaches a dangerous level. So, in addition to monitoring existing inflammation and adapting the light treatment using devices and systems of various representative embodiments, the present teachings contemplate monitoring the change in temperature caused by the treatment, and adjusting the treatment to avoid deleterious impact on the tissue.

As alluded to above, the Fdots 202 are selected from a number of materials adapted to cause fluorescent emission at a particular wavelength, and to terminate emission in the presence of a particular RNS or ROS. As will be appreciated, depending on the material chosen for the Fdots, the "termination" of emission may vary. For example, in some representative embodiments, emission stops completely and the Fdots function as an on-off switch. In other representative embodiments, the emission reduces gradually or partly for example by including Fdots comprising different materials that can impact the spectra, sensitivity and specificity to provide a gradual output signal from the Fdots. More generally, the degree of termination of fluorescent emission is related linearly or non-linearly to the concentration of NO. Notably, the correlation between output (emission) signal and NO concentration may not always be initially obvious, and certain transformations and calculations may be required to get to relation between fluorescent emission signal and NO concentration.

Moreover, in other representative embodiments, depending on the material chosen, the Fdots are chosen to fluoresce in the presence of RNS or ROS. Again, depending on the material chosen for the Fdots, the initiation of emission will vary. For example, in some representative embodiments, emission starts immediately and the Fdots function as an on-off switch. In other representative embodiments, the emission starts gradually. More generally, the degree of commencement of fluorescent emission may also be related to the concentration of NO. As such, by the selection of materials used for the Fdots, NO concentration can be made to gradually change fluorescence (emission) intensity, but also other factors including reaction time may play a role. In many applications such as described more fully below, the excitation wavelength or the emission can be selected for treating tissue with light therapy.

In certain applications, the Fdots comprise Qdots. Quantum dots are nanoscale crystals with optical and electric properties. They can emit or absorb specific wavelengths. Qdot biosensors are developed as fluorescence sensor for NO. The sensor emits fluorescent light when excited by visible (e.g., blue) light and turns off fluorescence when NO is present meaning the Qdots will code for a visual signal response in the presence of NO. Like other types of Fdots, Qdots comprise a material having a valence band and a conduction band separated by an energy gap that depends on the nature and the size of the material. After the absorption of light having a particular wavelength/energy, the electrons are excited to the conduction band, leaving a hole in the valence band. The photo-generated electron-hole pair is known as an exciton, which, upon recombination, gives rise to the fluorescence emission.

One drawback to certain known Qdot materials is their inclusion of heavy metals. Heavy metals can be toxic, and as such may not be useful in applications to internal tissues (e.g., the gums). As such, in certain applications, Qdots materials comprising heavy metals cannot be used. However, in other applications, the material 204 is selected to encapsulate the Qdot material comprising the heavy metal in a biocompatible material (e.g., polymer or silicone) that are beneficially permeable to NO to render its use safe in internal applications.

Qdots comprising heavy metals may, however, be used in applications that are not internal, such as the skin. In such applications, the Qdots usefully provide an indication of the presence and concentration of NO, which in turn is indicative of inflammation caused by a skin disease or injury.

Qdots that do not comprise heavy metals are also contemplated. These Qdots are generally not toxic and therefore may be used in internal applications, such as mouthpiece 201. Qdots contemplated for use in such internal applications comprise, but are not limited to Silicon Qdots, Carbon Qdots and Graphene Qdots. These materials are nanocrystals embedded in a polymer matrix (e.g., material 204)

Silicon Qdots lend greater biocompatibility and provide certain optical properties, such as long-lived excited states, large Stokes shift and tunable luminescence that can be beneficial in certain applications.

Carbon Qdots also lend greater biocompatibility due to their nontoxicity, while providing comparatively greater optical absorptivity, chemical stability and comparatively easy synthesis. Like Silicon Qdots, the fluorescence of Carbon Qdots is more efficient than many other materials, and thus lends itself to improved detection of NO, treatment of inflamed tissue, and monitoring of the progress of treatment of tissue.

Graphene Qdots when properly treated can also be used in vivo. Graphene Qdots comprise a layer of graphene and are chemically and physically comparatively stable. Graphene Qdots exhibit fluorescence emission across a comparatively large spectral range, including UV radiation, visible light an infrared (IR) radiation.

Just by way of illustration and not limitation, the Qdots may comprise a fluorescent molecularly engineered material comprising with iron(III) dithiocarbamates; Cadmium Selenium-Zinc Sulfur (CdSe-ZnS) nanocrystals; and surface bound tris(N-(dithiocarboxy)sarcosine)iron(III).

By way of illustration and not limitation, "turn-on" Qdots may comprise CdSe-ZnS nanocrystals as fluorophores and surface bound tris(N-(dithiocarboxy)sarcosine)iron(III) as reactive centers for NO.

In certain representative embodiments, both Qdots that emit light in the presence of NO and Qdots that terminate emission in the presence of NO are contemplated. These Qdots are referred to as "turn-on/turn-off' Qdots. By way of illustration and not limitation, the following materials are contemplated for use as "turn-on/turn-off' Qdots useful in the detection of NO: Fe⁺³; group II materials (alkyl metals, metal oxides or organic salts) with the use of an inorganic shell (e.g., ZnS); and group VI materials (Se, S and Te) with the use of an inorganic shell (e.g., ZnS).

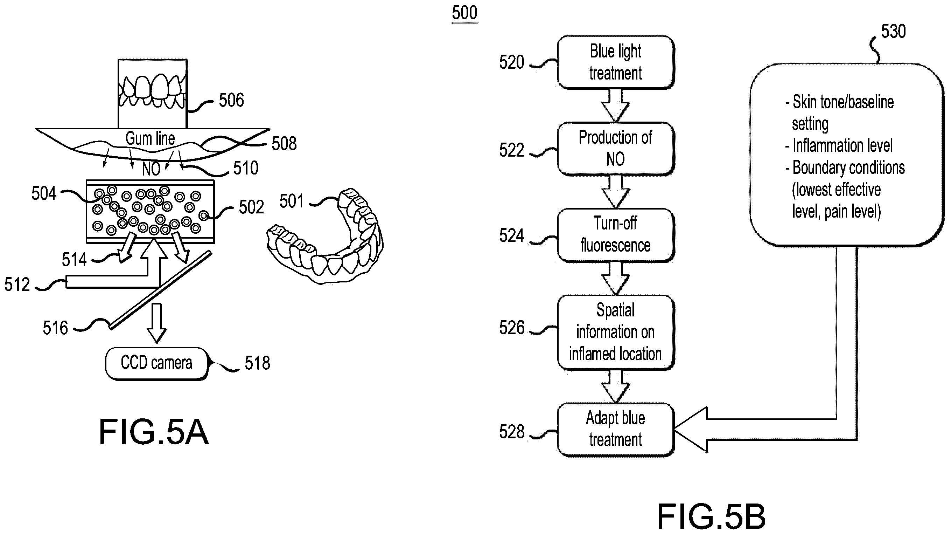

As described more fully below, once detected, the areas of the skin where inflammation exists can be treated with light of a particular wavelength useful in treating the underlying condition. Alternatively, or additionally, and again, as described more fully below, the Fdots may be used to monitor the progress of treatment of a condition. Specifically, as the treated tissue heals, the inflammation is reduced, and the resulting production of NO decreases. As the presence of NO decreases, when the Fdots are "turn-off' Fdots, the fluorescent emission caused by the excitation light source increases. By contrast, when "turn-on" Fdots are used, as the treated tissue heals, the inflammation is reduced, and the production of NO decreases. As the presence of NO decreases, when the Fdots are "turn-on", the fluorescent emission caused by the excitation light source decreases. Moreover, in certain representative embodiments such as described below in connection with Figs. 5A-5B, the Fdots emit light of one wavelength when light of an excitation wavelength is incident thereon, and emits light of a different wavelength in the presence of NO.

In accordance with other representative embodiments, the Fdots 202 are polymer dots (Pdots). Just by way of illustration, sub fluorophore groups (groups such as C = O, C = N, N = O) are contemplated for use as Pdots in accordance with various representative embodiments. Unlike Qdots that may comprise toxic materials, Pdots comprise organic nanoparticles (e.g., semiconducting polymer nanoparticles (SPNs)) assembled from polymer chains with π-conjugated systems. Pdots may consist of hydrophobic semiconducting polymers with a volume or weight fraction more than 50% and a diameter generally less than 50 nm, sometimes the particles size can be less than 30 nm. Pdots have shown characteristics of large absorption cross section, high brightness, good photostability, low toxicity. Moreover, Pdots provide comparatively high fluorescence brightness under both one photon and two-photon stimulation, and therefore are useful in biosensing and treatment applications such as those described in connection with various representative embodiments described herein.

As described more fully below, certain Pdots emit fluorescent light when excited by visible blue or violet light and turn off fluorescence when nitric oxide (NO) is present. Moreover, certain Pdots emit fluorescent light when excited by visible blue or violet light and emit fluorescent light of a different wavelength (e.g., near infra-red (NIR) when nitric oxide (NO) is present. As such, Pdots will code for a visual signal response in the presence of NO.

As described more fully below, once detected, the areas of the skin where inflammation exists can be treated with light of a particular wavelength useful in treating the underlying condition. Alternatively, or additionally, and again, as described more fully below, the Pdots may be used to monitor the progress of treatment of a condition. Specifically, as the treated tissue heals, the inflammation is reduced, and the production of NO decreases. As the presence of NO decreases, the fluorescent emission caused by the excitation light source increases. Alternatively, in certain other representative embodiments, when the presence of NO decreases, the fluorescent emission decreases.

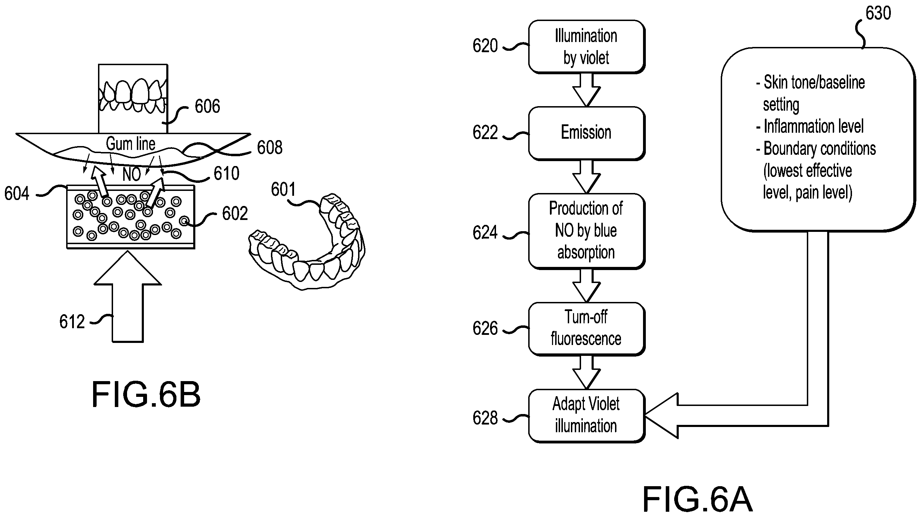

Fig. 2B is a graph showing Fdot size versus emission wavelength. As alluded to above, different Fdot materials have different excitation and emission wavelengths. Moreover, the size of the Fdots varies depending on the material selected. As shown in Fig 2B, the emission wavelength increases with increasing Fdot size (volume). As described more fully below, in certain applications it is useful to select the wavelength used for treatment, and/or the excitation wavelength, and/or the emission wavelength. As such, by the present teachings, the size of the Fdot as a function of emission wavelength is useful. Just by way of illustration, when the same treatment wavelength for tissue is assumed, the size of the Fdots used in the representative embodiments of Figs. 5A-5B are smaller than the size of the Fdots used in the representative embodiments of Figs. 6A-6B.

Furthermore, and as will be appreciated from a review of Fig. 2B, the size of the Fdots contemplated for use in accordance with devices of the various representative embodiments are comparatively small. The Fdot sensors size lend easily for adaptation into mouthpieces or other devices for medical diagnosis and treatment. As such, the level of response can be collected by integrated sensors that are small enough to be captured by the mouthpiece comprising a plurality of Fdots is improved compared to certain known devices and systems.

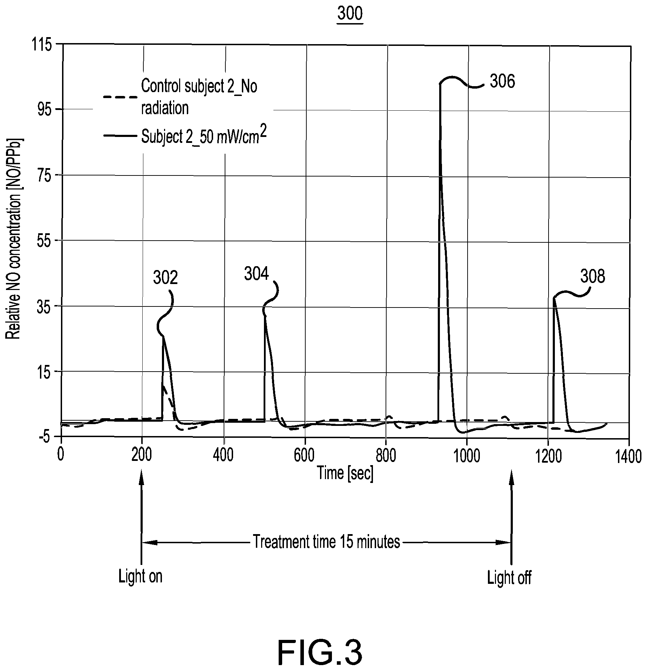

Fig. 3 is a graph showing onset of NO production in the presence of blue light having a wavelength of 450 nm versus time. Specifically, the blue light is applied to the tissue for a period of time, and the resultant peaks of NO are detected. First curve 302, second curve 304, third curve 306 and fourth curve 308 are measures of the concentration of NO at times of sampling with the fourth curve 308 indicative of the cessation of illumination has stopped, and shows that NO is still released from skin for a time after illumination is terminated. Consequently, as described more fully below, light-induced NO production can serve as a `memory system' of treated positions of tissue and according to various representative embodiments, light treatment can be adapted and targeted to aim for better safety and effectiveness.

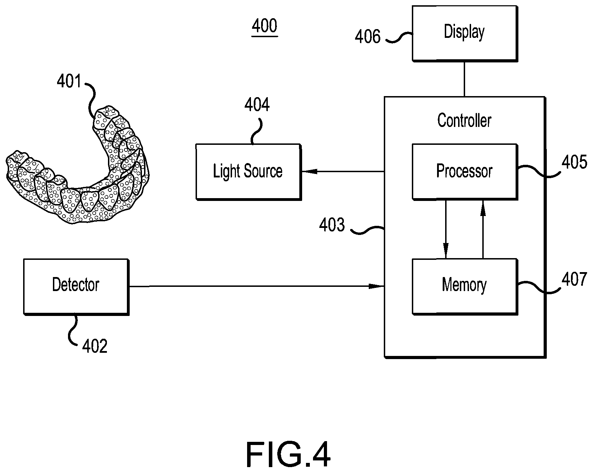

Fig. 4 is a simplified block diagram of a system 400 for evaluating and/or treating oral tissue using an inventive mouthpiece, in accordance with a representative embodiment. Various aspects and details of presently described system may be common to those described in connection with representative embodiments above, and may be used in systems of representative embodiments described below. These common aspects and details may not be repeated in order to avoid obscuring the presently described representative embodiments.

The system 400 comprises a mouthpiece 401, which comprises a plurality of Fdots disposed in a suitable material. It is emphasized the mouthpiece 401 is merely an illustrative device, and other devices are contemplated for use in connection with the system 400. Moreover, these alternative devices are contemplated for both internal and external use with appropriate materials and devices based on the application. Just by way of illustration, and as noted below, instead of the mouthpiece 401, the device may be adapted to monitor and/or treat skin conditions that are responsive to light treatment including, for example inflammatory skin diseases or injuries such as overexposure to UV radiation.

The system 400 comprises a detector 402. As will be appreciated, the detector 402 is selected to detect the presence of RNS or ROS. In certain embodiments, the detector 402 is adapted to detect the presence of NO. In certain embodiments, the detector 402 is adapted to determine the location and time of the presence of NO. As described more fully below in connection with various representative embodiments, different types of detectors are contemplated. For example, in certain representative embodiments, the detector 402 comprises a pixelated (1D, 2D or 3D) charge coupled device (CCD) or a complementary metal oxide semiconductor (CCD) device. In other representative embodiments, the detector 402 detects NO by monitoring the chemiluminescence of the reaction of NO with ozone. In this reaction, NO₂ is formed in an excited state, which upon decay to the ground state emits a photon of blue light. In yet other representative embodiments, the detector 402 may comprise electrochemical sensors, including for example, but not limited to differential pulse voltametric (DPV) or diphenyl phosphate (DPP) electrochemical sensors. In yet other embodiments, the detector 402 may comprise a photoacoustic sensor.

The system 400 also comprises a light source 404. The light source 404 is selected to provide an excitation wavelength to cause fluorescent emission from the Fdots as described above, or to provide light of a selected wavelength for treatment of the tissue as alluded to above and as described more fully below. The light source 404 is also adapted to modify the intensity of light incident on the mouthpiece 401 and/or the tissue undergoing treatment, and/or the time of the application of light to the tissue, as described below.

The modification of the applied light from the light source 404 may carried out in a number of ways based on data the output from the detector 402. For example, in certain representative embodiments, data from the detector 402 is adapted enable measurements of NO concentration relative to a threshold. Illustratively, in accordance with a representative embodiment, based on data from the detector 402, the instructions cause the system 400 to determine the location where and/or the time when, a concentration of the NO is greater than a threshold. Based on this predetermined threshold, the instructions may cause the system 400 to adjust the intensity and/or duration of light from the light source.

Just by way of illustration, the threshold may be in a range of approximately 30 parts-per-billion (ppb) and approximately 300 ppb NO. Moreover, the threshold may be set to indicate the level of NO is not existent. For example, the NO may be deemed not present when the concentration of the NO is below approximately 20 ppb.

Furthermore, the adjustment of the light source 404 may be iterative in accordance with certain representative embodiments. For example, after an adjustment of the light source 404 based on the data from the detector, the location is a first location, the time is a first time, and the instructions further cause the system to determine a second location where and/or a second time when the concentration of the NO is greater than the threshold; and further adjust the output of the light source at the excitation wavelength based on the second location and/or the second time.

Data from the detector 402 are provided to a controller 403. The controller 403 comprises a processor 405, and a memory 407, which stores computer-executable instructions (code). In accordance with representative embodiments described more fully below, these instructions, when executed by the processor 405 carry out various functions of representative embodiments described herein. As such, the memory 407 may store a set of software instructions that can be executed by the processor 405 to cause the system 400 to perform some or all aspects of certain methods or computer-based functions described herein.

The controller 403 may be implemented by a computer that includes more elements than the controller 403 and memory 407 of Fig. 4. Notably, in accordance with a representative embodiment, the controller 403 may be remote to the system 400, and is adapted to control various aspects of the system 400 remotely via connections including both wired and wireless connections and protocols. In this sense, the controller 403, at least in part is a specialty or particular computer useful in controlling the system 400.

The controller 403 may operate as a standalone device or may be connected, for example, using a network to other computer systems or peripheral devices. In representative embodiments, the system 400 performs logical processing based on digital signals received via an analog-to-digital converter. The controller 403 can also be implemented as or incorporated into various devices, such as a workstation that includes a controller, a stationary computer, a mobile computer, a personal computer (PC), a laptop computer, a tablet computer, or any other machine capable of executing a set of software instructions (sequential or otherwise) that specify actions to be taken by that machine. The controller 403 can be incorporated as or in a device that in turn is in an integrated system that includes additional devices. In an embodiment, the controller 403 can be implemented in a device that also provides video or data communication. Moreover, the controller 403 may be connected to components of the system via a local wired interface such as an Ethernet cable or via a local wireless interface such as a Wi-Fi connection.

The processor 405 may be considered a representative example of a processor of the controller 403 and executes instructions to implement some or all aspects of methods and processes described herein. The processor 405 is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor 405 is an article of manufacture and/or a machine component.

The processor 405 is configured to execute software instructions to perform functions as described in the various embodiments herein. The processor may be a general-purpose processor or may be part of an application specific integrated circuit (ASIC). The processor may also be a microprocessor, a microcomputer, a processor chip, a controller, a microcontroller, a digital signal processor (DSP), a state machine, or a programmable logic device. The processor may also be a logical circuit, including a programmable gate array (PGA), such as a field programmable gate array (FPGA), or another type of circuit that includes discrete gate and/or transistor logic. The processor may be a central processing unit (CPU), a graphics processing unit (GPU), or both. Additionally, any processor described herein may include multiple processors, parallel processors, or both. Multiple processors may be included in, or coupled to, a single device or multiple devices.

The term "processor" as used herein encompasses an electronic component able to execute a program or machine executable instruction. References to a processor should be interpreted to include more than one processor or processing core, as in a multi-core processor. A processor may also refer to a collection of processors within a single computer system or distributed among multiple computer systems, such as in a cloud-based or other multi-site application.

"Memory" is an example of computer-readable storage media, and should be interpreted as possibly being multiple memories or databases. The memory or database may for instance be multiple memories or databases local to the computer, and/or distributed amongst multiple computer systems or computing devices. A computer readable storage medium is defined to be any medium that constitutes patentable subject matter under 35 U.S.C. §101 and excludes any medium that does not constitute patentable subject matter under 35 U.S.C. §101. Examples of such media include non-transitory media such as computer memory devices that store information in a format that is readable by a computer or data processing system. More specific examples of non-transitory media include computer disks and non-volatile memories.

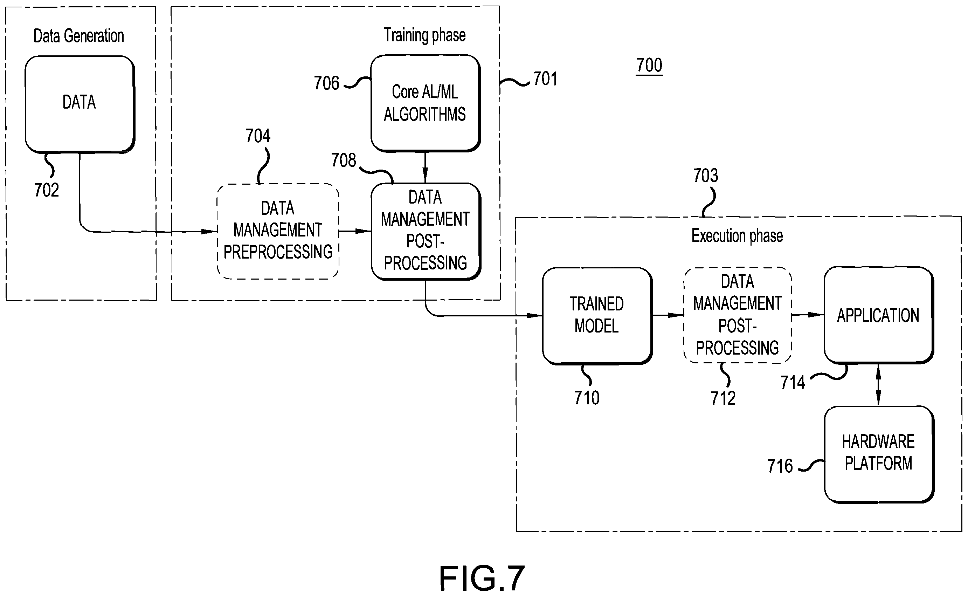

The memory 407 may include a main memory and/or a static memory, where memories in the system 400 communicate with each other and the processor via a bus (not shown). The memory may be considered a representative example of a memory of the controller 403, and store instructions used to implement some or all aspects of methods and processes described herein. Memories described herein are tangible storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory is an article of manufacture and/or machine components. The memory is a computer-readable medium from which data and executable software instructions can be read by a computer (e.g., by the processor of the controller 403). The memory may be implemented as one or more of random-access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, Blu-ray disk, or any other form of storage medium known in the art. The memory may be volatile or non-volatile, secure and/or encrypted, unsecure and/or unencrypted. The inventive concepts also encompass a computer readable medium that stores instructions that cause the processor to execute the methods described herein. Notably, the computational models described more fully below in connection with Figs. 7-10 are may be stored as instructions that cause the processor to execute the ML models of various representative embodiments. Finally, a computer readable medium is defined herein to be any medium that constitutes patentable subject matter under 35 U.S.C. §101 and excludes any medium that does not constitute patentable subject matter under 35 U.S.C. §101. Examples of such media include non-transitory media such as computer memory devices that store information in a format that is readable by a computer or data processing system. More specific examples of non-transitory media include computer disks and non-volatile memories.

Additionally, the memory 407 is an example of a computer-readable storage medium. Computer memory is any memory which is directly accessible to a processor. Examples of computer memory include, registers, and register files. References to "memory" should be interpreted as possibly being multiple memories. The memory may for instance be multiple memories within the same computer system. The memory may also be multiple memories distributed amongst multiple computer systems or computing devices. Software instructions, when executed by the processor, perform one or more steps of the methods and processes as described herein. In an embodiment, the software instructions may reside all or in part within the memory and/or the processor during execution by the controller 403.

The system 400 may also comprise a display 406 connected to the controller 403. In certain representative embodiments, the display 406 comprises a graphic user interface (GUI) and is connected to the controller 403 via a local wired interface such as an Ethernet cable or via a local wireless interface such as a Wi-Fi connection. The display 406 may be a monitor such as a computer monitor, a display on a mobile device, an augmented reality display, a television, an electronic whiteboard, or another screen configured to display electronic imagery.

The controller 403 may be interfaced with other user input devices by which users can input instructions, including mouses, keyboards, thumbwheels and so on. The controller 403 may also include one or more input interface(s) such as those noted above that may connect to other elements or components, as well as an interactive touch screen configured to display prompts to users and collect touch input from users. These interfaces may also be icons (not shown) on a display (not shown in Fig. 4 via the GUI).

As described more fully below, the display 406 enables the user to determine the presence and/or levels of RNS (e.g., NO) based on data from the detector 402, and enables the user (e.g., using the GUI or other interface) to provide inputs from the controller 403 to the light source 404. Just by way of illustration, and as described more fully below, the inputs provided to the light source may alter the duration, or location, or intensity, or a combination thereof, of light applied by the light source 404 based on data from the detector 402. Moreover, and again as described more fully below, based on the data received from the detector 402, the instructions in the memory 407 may cause the processor to alter the duration, or location, or intensity, or a combination thereof, of light applied by the light source based on data from the detector.

Fig. 5A is a conceptual view of a system 500 for evaluating and/or treating oral tissue using an inventive device in accordance with a representative embodiment. Various aspects and details of presently described system may be common to those described in connection with representative embodiments above, and may be used in systems of representative embodiments described below. These common aspects and details may not be repeated in order to avoid obscuring the presently described representative embodiments.

Turning to Fig. 5A, a mouthpiece 501 is shown for reference, and is used to monitor and/or treat gums 506 of a subject's mouth. A portion of the mouthpiece is enlarged to show the Fdots 502 disposed in a material 504 as shown. It is again emphasized that application of the teachings to oral healthcare is merely illustrative, and other applications are contemplated. Just by way of example, rather than gums 506 of a subject's mouth, the portion comprising Fdots 502 in material 504 may be crafted to be used to monitor other internal tissue, or external tissue, such as skin tissue.

As shown, light 512 is incident from a light source (not shown in Fig. 5A) on a wavelength dependent beamsplitter 516. The wavelength dependent beamsplitter 516 is adapted to reflect the light 512 towards the portion. In the presently described representative embodiment, the light 512 has a first wavelength selected to treat the tissue by transmission through the material 504, and to cause fluorescent emission 514 from the Fdots at second wavelength, which is different from the first wavelength. As such, in the present example, the first wavelength is the excitation wavelength because it causes fluorescent emission, and is selected to treat the inflammation in the tissue as described above. In certain representative embodiments, the excitation wavelength of the light 512 is violet or blue visible light, although other wavelengths are contemplated to carry out the excitation of the Fdots while at the same time providing the therapeutic light treatment of the particular tissue and the particular cause of the inflammation.

As shown, NO 510 emanates from the tissue (e.g., gums 506) and traverses the portion comprising the Fdots 502 disposed in the material 504. As described above, in the presence of NO, the fluorescent emission 514 by the Fdots 502 terminates, and as described below, this termination of fluorescent emission 514 by the Fdots 502 allows the locations of NO production resulting from inflammation and/or non-inflammatory processes such as from light therapy on the tissue to be determined, and the time at which the inflammation exists, as may be desired.

The wavelength of the fluorescent emission 514 traverses the wavelength dependent beamsplitter 516 unimpeded, and is incident on a charge-coupled device (CCD) camera 518. As such, in the present example, the second wavelength is the emission wavelength. As noted above, the Fdots 502 are selected to comprise a material that emits fluorescent radiation having a first wavelength when light of the excitation wavelength is incident thereon, and emits fluorescent radiation of a second (different) wavelength in the presence of NO. In certain representative embodiments, the emission wavelength of the fluorescent emission 514 is near-infrared (NIR), although other wavelengths are contemplated to carry out the excitation of the Fdots.

As shown, the fluorescent emission 514 is incident on the CCD camera, which identifies the regions where and when NO is present, but also by where and when NO is absent. Specifically, the CCD camera records areas/times of the gums 506/tissue where light of the emission wavelength is present, and by absence where areas/times where the light of the emission wavelength is absent. Stated somewhat differently, light 512 is adapted to be incident on particular portion of the gums 506/tissue being monitored and/or treated. In regions of the portion of the gums 506 where NO is present, the fluorescent emission 514 terminates, and in regions where NO is not present, the fluorescent emission 514 continues caused by the light 512. These data are recorded, and provided to the controller 403 so the processor 405 can alter the output of the light source to provide light 512 to treat regions where the inflammation of the tissue exists. Moreover, and as alluded, these data from the CCD camera 508 can also be used to monitor the progress of treatment of the tissue by the light 512. Based on these data, the processor 405 can alter the output of the light source so that light of a particular intensity is applied for a particular time to provide the needed light treatment. Finally, these may also be used to monitor the temperature of the tissue under treatment based on.

Fig. 5B is a flow chart of method for evaluating and/or treating oral tissue using an inventive device in accordance with a representative embodiment. Various aspects and details of presently described system may be common to those described in connection with representative embodiments above, and may be used in systems of representative embodiments described below. These common aspects and details may not be repeated in order to avoid obscuring the presently described representative embodiments.

The method begins at 520 with the application of light 512 from the light source. At 522 NO production exists. As noted above, the NO production may arise not only from inflammation in the tissue being treated, but also from the applied light. At 524 fluorescence initiates due to the presence of the NO.

At 526, data from the CCD camera are used to determine spatial (and if desired) temporal aspects of the inflammation. As noted above, based on these data, through execution of instructions stored in memory 407, the processor 405 can alter the output of the light source so that light of a particular intensity is applied for a particular time to provide the appropriate light treatment. Moreover, these data from the CCD camera 508 can also be used to monitor the progress of treatment of the tissue by the light 512. Finally, these data may also be used to monitor the temperature of the tissue under treatment as noted above.

Table 530 lists various parameters that can be stored in memory 407 to select the appropriate intensity and duration of the treatment. These factors include, but are not limited to, skin tone/baseline levels, inflammation level and boundary conditions. In accordance with a representative embodiment, these parameters stored in a look-up table in memory, and based on the NO levels and locations, adjustments of the treatment light are carried out. So, just by way of illustration, in accordance with a representative embodiment, for a given set of skin tone/inflammation/boundary conditions stored in memory (e.g., in a look-up table), a light intensity level and duration are assigned, and through execution of instructions stored in memory, the processor 405 causes the light source to adjust the location, and/or intensity, and/or duration of the treatment light applied. In accordance with another representative embodiment, locations where a subject indicates a location a certain symptom (e.g., sore or swollen gums) may be provided (e.g., via the GUI or other interface). Based on this information, at 528 the processor 405 causes the light source to adapt the location and/or intensity to ensure a particular treatment is carried out at a particular. Moreover, subject-specific data (e.g., pain threshold) can be stored in memory, and execution of instructions by the processor 405 can cause the adaptation of the light source to carry out a treatment having an appropriate intensity and duration based on the subject-specific data.

Fig. 6A is a conceptual view of a system for evaluating and/or treating oral tissue using an inventive device in accordance with a representative embodiment. Various aspects and details of presently described system may be common to those described in connection with representative embodiments above, and may be used in systems of representative embodiments described below. These common aspects and details may not be repeated in order to avoid obscuring the presently described representative embodiments.

Turning to Fig. 6A, a mouthpiece 601 is shown for reference, and is used to monitor and/or treat gums 606 of a subject's mouth. A portion of the mouthpiece is enlarged to show the Fdots 602 disposed in a material 604 as shown. It is again emphasized that application of the teachings to oral healthcare is merely illustrative, and other applications are contemplated. Just by way of example, rather than gums 606 of a subject's mouth, the portion comprising Fdots 602 in material 604 may be crafted to be used to monitor other internal tissue, or external tissue, such as skin tissue.

As shown, light 612 is incident from a light source (not shown in Fig. 6A) on Fdots 602 disposed in material 604. The light 612 has a wavelength selected to cause fluorescent emission 614 from the Fdots 602. As such, in the present example, the light 612 is the excitation wavelength because it causes fluorescent emission 614. In this representative embodiment, the wavelength of light of the fluorescent emission 614 is blue and is selected to treat the inflammation in the tissue as described above. In certain representative embodiments, the excitation wavelength of the light 612 is violet, although other wavelengths are contemplated to carry out the excitation of the Fdots 602 while at the same time providing the therapeutic light treatment of the particular tissue and the particular cause of the inflammation.

As shown, NO 610 emanates from the tissue (e.g., gums 606 and traverses the portion comprising the Fdots 602 disposed in the material 604. As noted above, the Fdots 602 are selected to comprise a material that not only fluoresces when light of the excitation wavelength is incident thereon, but also to terminate fluorescent emission by the Fdots 602 in the presence of NO when turn-off Fdots are implemented, or initiate fluorescent emission in when turn-on Fdots are implemented.

In accordance with a representative embodiment, the detector 402 is adapted to determine the level of NO in the region around the tissue. These data are recorded, and provided to the controller 403 so the processor 405 can alter the output of the light source to provide light 612 to treat regions where the inflammation of the tissue exists. Moreover, and as alluded, these data can also be used to monitor the progress of treatment of the tissue by the light 612. Based on these data, the processor 405 can alter the output of the light source so that light of a particular intensity is applied for a particular time to provide the needed light treatment. Finally, these data may also be used to monitor the temperature of the tissue under treatment as noted above.

Fig. 6B is a flow chart of method for evaluating and/or treating oral tissue using an inventive device in accordance with a representative embodiment. Various aspects and details of presently described system may be common to those described in connection with representative embodiments above, and may be used in systems of representative embodiments described below. These common aspects and details may not be repeated in order to avoid obscuring the presently described representative embodiments.

The method begins at 620 with the application of light 612 from the light source. At 622 NO production exists. As noted above, the NO production may arise not only from inflammation in the tissue being treated, but also from the applied light. At 624 fluorescence terminates due to the presence of the NO caused by inflammation and/or absorption of blue light.

At 626, data from the detector 402 are used to determine concentration of NO near the tissue being treated, and based on these data, the degree of inflammation can be determined. As noted above, based on these data, through execution of instructions stored in memory 407, at 628 the processor 405 can alter the output of the light source so that light of a particular intensity is applied for a particular time to provide the appropriate light treatment. Moreover, these data from the detector can also be used to monitor the progress of treatment of the tissue by the light 612. Finally, these data may also be used to monitor the temperature of the tissue under treatment as noted above.

Table 630 lists various parameters than can be stored in memory 407 to select the appropriate intensity and duration of the treatment. These factors include, but are not limited to, skin tone/baseline levels, inflammation level and boundary conditions. In accordance with a representative embodiment, these parameters stored in a look-up table in memory, and based on the NO levels and locations, adjustments of the treatment light are carried out. So, just by way of illustration, in accordance with a representative embodiment, for a given set of skin tone/inflammation/boundary conditions stored in memory (e.g., in a look-up table), a light intensity level and duration are assigned, and through execution of instructions stored in memory, the processor 405 causes the light source to adjust the location, and/or intensity, and/or duration of the treatment light applied. In accordance with another representative embodiment, locations where a subject indicates a location a certain symptom (e.g., sore or swollen gums) may be provided in the GUI or other interface. Based on this information, the processor 405 causes the light source to adapt the location and/or intensity to ensure a particular treatment is carried out. Moreover, subject-specific data (e.g., pain threshold) can be stored in memory, and execution of instructions by the processor 405 can cause the adaptation of the light source to carry out a treatment having an appropriate intensity and duration.

In accordance with representative embodiments described more fully below, adaptation of light at 528, 628 is done to carry out a treatment using a trained computational model. These computational models (ML algorithms) are adapted to predict a health status of tissue, predict treatment settings (e.g., blue light treatment at a certain intensity and for a certain duration), and predict the NO response of the tissue to the treatment. To this end, as described above, there are known relations between NO production and tissue health. Both the health of tissue and NO production by the tissue are influenced by factors such as light and/or drug treatment, and environmental factors like temperature. Accordingly, for a particular individual, the tissue health, treatment settings and NO response by the tissue as a result of the treatment at the predicted treatment settings are based on the knowledge that NO is an indicator of tissue health and changes in NO indicated changes in health, with the rate of changes dependent on multiple factors such as selected treatment and environment. Various representative embodiments described below are directed to using ML algorithms to predict not only the health of the tissue, but also treatment settings based on the health of the tissue and their impact on the health of the tissue. Finally, and as will become clearer as the present description continues, through the ML algorithms of the various representative embodiments, the health status or diagnosis of tissue, the selection of suitable treatment settings, and the resultant NO response to the treatment can be beneficially carried out in a comparatively reduced amount of time when compared to other known iterative ways of diagnosing the health and determining the proper settings to realize a desired treatment.

Fig. 7 is a conceptual diagram 700 various aspects of a computational model for predicting tissue treatment settings and (NO) response of tissue in accordance with a representative embodiment.

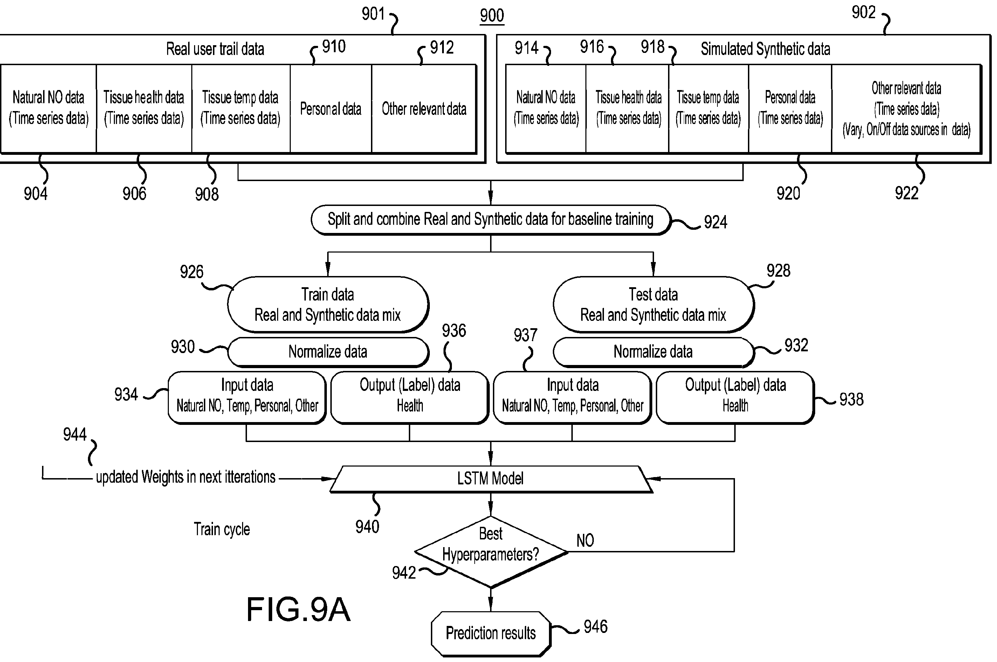

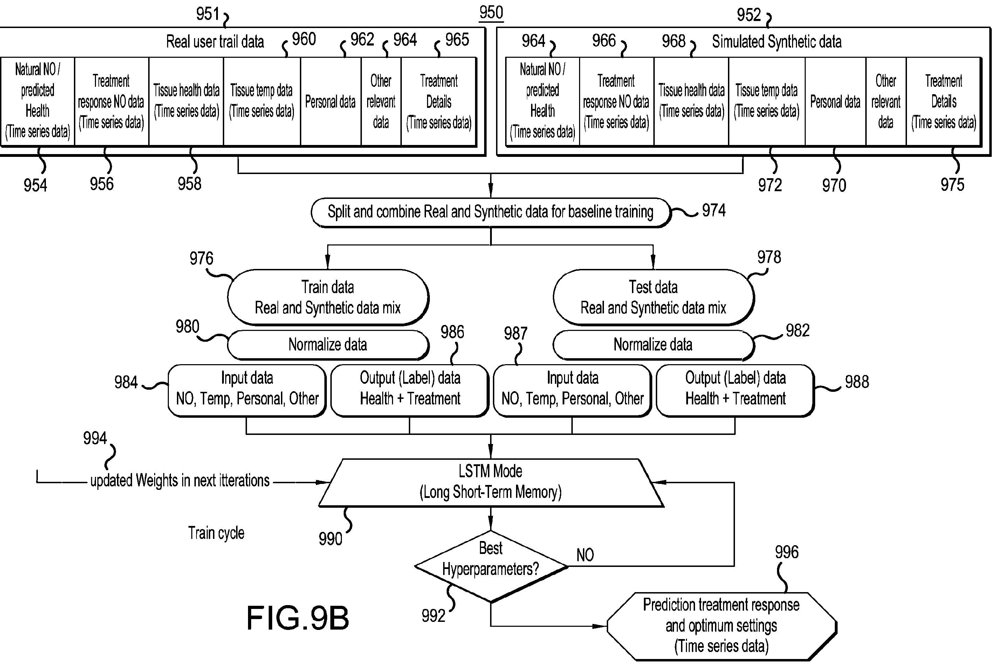

In baseline training phase 701, a baseline ML model is realized. Data 702 are gathered and stored in memory 407 or other suitable memory. As described more fully below in connection with Figs. 9A and 9B, these data comprise one or more of natural NO data and predicted health status data, treatment response NO data, tissue health data, tissue temperature data, personal data, and treatment details for individual and a large pool of other individuals. Notably with the exception of the static personal data, these data are time-series data, which are a sequence of data points collected over time at consistent intervals, allowing analysis of how a variable changes over a specific period, often revealing patterns, trends, and seasonal fluctuations within the data. Data 702 also comprises "real" data and synthetic data. As described more fully below, the real data comprise ground truth data, and the synthetic data comprise data garnered from a simulation model. Notably, the simulation model is based on known modeling of natural human body system behavior relevant for this specific case. Notably, the synthetic data are optional, and are generally included in training the model to provide a sufficient amount of data to aid in generating a substantially reliable ML algorithm.

At 704, data management and preprocessing of the data 702 is carried out. The preprocessing is done to split and combine the real data and the synthetic data. The splitting provides a training set of data and a test set of data, whereas the combining of data provides both real and synthetic data in the training and test sets of data. As described more fully below, the preprocessing follows a series of steps to provide suitable inputs for the ML model to provide health status, and/or tissue treatment settings and (NO) response of tissue.

The training phase also comprises core ML algorithms 706 that are selected to provide a suitably accurate trained model for carrying out the methods by the systems of the various representative embodiments. In accordance with certain representative embodiments, the core ML algorithm comprises a Long Shorty Term Memory (LSTM) model. Notably, the LSTM model is merely illustrative, and other ML algorithms are contemplated for use in accordance with various representative embodiments. LSTM models are configured to process and analyze sequential data, such as time series, text, and speech. LSTM models may use a memory cell and gates to control the flow of information, allowing the model to selectively retain or discard information as needed and thus avoid the vanishing gradient problem that plagues traditional recurrent neural networks (RNN). It is noted that the use of LSTM models is merely illustrative, and other ML models are contemplated for use in connection with various representative embodiments described herein.

Training, data management and post-processing at 708 comprises baseline training the LSTM model to predict the health of the tissue and/or the tissue treatment settings and (NO) response of tissue at point in time (t0) and the predicted changes at a later point in time (t0+n). As described more fully above, there is a relation between NO production and tissue health with variations per person and over time, and both tissue health and NO production are influenced by factors such as light and/or drug treatment, and environmental factors like temperature. The predictions of the health status and/or the tissue treatment settings and (NO) response of tissue are based on the fact that NO emission is an indicator of the health status of tissue and that changes in NO emissions indicate changes in health of the tissue. Moreover, the rate of changes in NO emissions are dependent on factors such as treatment settings and duration, the environment, and as such are different for every individual.

The LSTM model uses labeled time series data of a mix of populations representing a variety of individuals in this baseline training (training phase 701) with the data used in baseline training comprising tissue health (e.g., NO emissions, tissue temperature, and personal data such as age, gender). In addition, other data may be optionally used in the baseline model training including humidity, medication, know related medical conditions of the individuals that comprise the data pool (data 702) used in the baseline training. Moreover, safety limits, which are described more fully below may also be used as inputs used by the algorithm baseline training.

While training the LSTM model at 708, weights of the model may be updated with the aim of beneficially minimizing the loss. Illustratively, loss may be minimized using a mean squared error (MSE) calculation. The MSE calculation is often useful default loss calculation used in regression calculations. MSE is calculated as the average of the squared differences between the predicted and actual values. The result is always positive regardless of the sign of the predicted and actual values and a perfect value is 0.0. The squaring results in comparatively larger miscalculations and greater error than comparatively smaller miscalculations. As such, larger mistakes are thus more costly to the model during training. LSTM is a general example model and offering a variety of different loss functions.

A baseline trained model 710 (sometimes referred to herein as the "trained model") is deployed in an execution phase 703. As such, the LSTM model of certain representative embodiments predicts the health of tissue and/or predict tissue treatment settings and (NO) response of tissue as described more fully below.

At 712, data management post-processing is carried out. Generally, data management post-processing comprises verifying whether the predicted values are within safety limits and/or other required boundaries. Moreover, the output of the model, the model and model weights can be optimized in format and shape before deployment, and integrated into the deployed systems, such as systems with different resources available or different treatment capabilities.

At 714, the trained model is deployed and predicts the health of tissue and/or predict tissue treatment settings and (NO) response of tissue as described more fully below.

As described more fully below, after execution of the model, for individual training the trained model may be used in a transfer training approach where the baseline model is updated or tuned based on the personal use. This updating involves verifying calculated predictions in time with actual NO readings. Moreover, and as described more fully below, the ML algorithm of representative embodiments in which treatment settings are predicted include providing an added safety and training step using the safety check in the treatment predictions as another input for the model. Notably, this may be done not only in baseline training, but also in all aspects of training according to the present teachings, thereby providing a further safety check and an important training input. If the predicted treatment settings are too close to or over the safety limit. Just by way of illustration, the safety limits may comprise legal (e.g., U.S. Food and Drug Administration (FDA) safety limits, or safety limits based on known higher risk levels for specific users, treatment or medication combinations. By including such limits in the training of the ML model, the predicted treatment may be corrected and the model training may be updated to avoid providing wrong unsafe values.

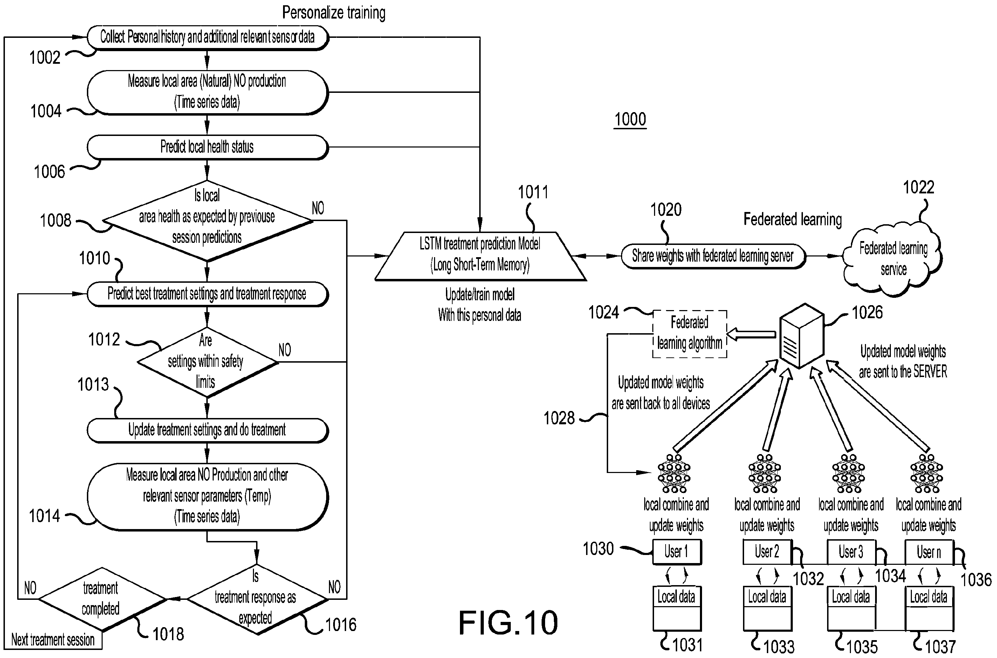

Finally, and as described more fully below in connection with Fig. 10, federated learning (FL) may be used to share model weights of actual users to further improve the ML algorithm during deployment. Federated learning updates aspects of the model for individual deployments as well as updates the baseline for new deployments. At a tunable rate, weights are collected from users in a central server, from these weights a general weight can be calculated / updated.

In FL of a representative embodiment, a weighted aggregation of local models may be conducted to generate a global model, and the aggregation weights are normalized (the sum of weights is 1) and proportional to the local data sizes. The influence of the federated learning on the individual trained models can be tuned, depending on the personal influence on the algorithm/weights and the quality of how well the baseline trained model matches the user(s). Moreover, federated users may be divided geographically or based on other criteria to provide further refine and more accurate models by federated learning influence.

Finally, at 716 the hardware platform useful for the type of measurements taken is improved. This hardware, which comprises for example the mouthpiece adapted to sense NO emissions, can be improved by adjustment (e.g., scaling) for a particular individual.

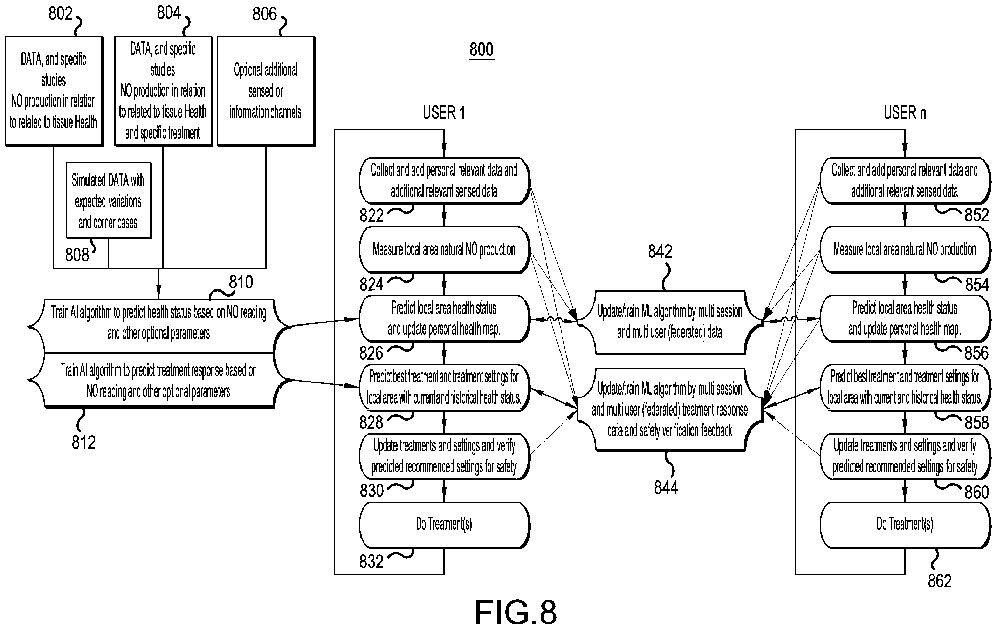

Fig. 8 is a flow-diagram 800 of baseline training of a computational model for predicting a health status of tissue, and/or tissue treatment settings and (NO) response of tissue, execution of the computational model, and updating of the model, in accordance with a representative embodiment. Certain aspects and details of the conceptual diagram described above in connection with representative embodiments may be common to the description of the flow-diagram 800. These common aspects and details may not be repeated to avoid obscuring the presently described representative embodiments.

Data 802 comprise specific data of NO production and the health status of tissue from a comparatively large group of individuals and from specific studies. These data may be referred to herein as ground truth data. As described more fully below in connection with Fig. 9A, these data are used in baseline training of a ML model adapted to predict the health status of an individual.

Data 804 comprise specific data of NO production resulting from treatment at particular treatment settings from a comparatively large group of individuals and from specific studies. These data may be referred to herein as ground truth data. As described more fully below in connection with Fig. 9B, these data are used in baseline training of a ML model adapted to predict treatment settings (e.g., settings of light source 404) and the predicted NO response of the tissue.

Data 806 comprise optional additional sensed or information channels and may comprise additional data including, but not limited to personal health data, temperature, medication, age, treatment (effect) history, different treatment embodiments, any additional data, possible in combination with the NO data that can have direct or indirect influence on the (predicted) health / NO reading. These data 806 also include a location and type of local tissue correlated to the data, which can be used in the preparation of a health map as described more fully below.

Data 802, 804, 808 are provided to an ML algorithm 810 being trained to predict the health status of tissue based on NO measurements and other optional parameters such as hyperparameters. Illustrative hyperparameters may comprise learning rate, momentum, batch size, Epochs, and dropout settings used in tuning the model, as described more fully below. As is also described more fully below in connection with Fig. 9A, the training of ML algorithm 810 results in a trained model for an individual, which when executed predicts the health of tissue based on NO data acquired from the individual.

Data 802, 804, 808 and 806 are provided to a ML algorithm 812 being trained to predict the treatment settings and the NO response of the treatment based on measurements and other optional parameters such as those as noted above As described more fully below in connection with Fig. 9B, the training of ML algorithm 810 results in a trained model for an individual, which when executed predicts tissue treatment settings (e.g., of light source 404) and (NO) response of tissue based NO data acquired from the individual.

At 852, execution of the ML model begins with collection of personal data from an individual being tested, as well as other relevant data (e.g., as noted above including age, medication, history) related to the individual.

At 854, NO production is measured. Notably, at 854 both the naturally occurring NO production (e.g., cause by inflammation and/or healing of the tissue at a particular time and at a particular location) of the tissue is measured. Moreover, stimulated NO production caused by light treatment is measured. These measurements may be taken using a personal healthcare device comprising Fdots (e.g., a mouthpiece or a bandage) that is disposed adjacent to the tissue being evaluated.

At 856, the trained (baseline) ML model from ML algorithm 810 is applied to the data gathered at 854 and is used to predict the health status of the tissue at the particular time and location of the tissue where NO data are gathered. Notably, in certain representative embodiments, the determination of the health status of the tissue is the sole objective of execution of the method. In this case, the results of 856 are provided to the model, and at 842 an updating of the ML model is carried out. The updating of the ML model at 842 comprises updating of the weights of the model for the individual, and may include sharing of weights and parameters based on received federated learning rates, as described more fully in connection with Fig. 10.

As described more fully below, in accordance with a representative embodiment, a health map of the tissue of the individual can be made and updated at 856. In accordance with a representative embodiment, the ML algorithm 812 is applied iteratively at the particular location at other times. Based on the predicted health status of the tissue at the particular location, the health map can be generated. As described more fully below, an area in the health map that is related to the particular location is used to update the weights of the model at the particular location for the individual.

At 858, the trained (baseline) model from ML algorithm 812 is applied to the data gathered at 854 and to current and historical health status of the individual. The ML algorithm 812 then predict tissue treatment settings and thereby the recommended treatment. The resultant NO tissue response of the predicted treatment settings/treatment are also predicted at 858.

In this case, the results of 858 are provided to the model, and at 844 an updating of the ML model is carried out. The updating of the ML model at 842 comprises updating of the weights of the model for the individual, and may include sharing of weights and parameters based on received federated learning rates, as described more fully in connection with Fig. 10.

At 830, updating of the ML algorithm 812 is carried out. The predicted treatment settings predicted are then compared to a safety limit. As described more fully below, when the comparing of the predicted treatment settings is equal to or below the safety limit, the updated predicted treatment settings are stored and a tissue treatment at the predicted treatment settings can be carried out. However, when the comparison of the predicted settings is greater than the safety limit, treatment settings are adjusted by updating weights of the trained computational model and the iterative prediction of treatment settings and comparison to the safety limit is done until the predicted treatment settings is equal to or below the safety limit.

At 832, the treatment at the predicted treatment settings is carried out for the individual.

As shown in Fig. 8, the application of the trained ML algorithm is repeated for another individual (user n). At 852, execution of the ML model begins with collection of personal data from an individual being tested, as well as other above-noted relevant data related to the individual.

At 854, NO production is measured. Notably, at 854 both the naturally occurring NO production (e.g., caused by inflammation in the healing of tissue as noted above) at a particular time and at a particular location of the tissue is measured. Moreover, stimulated NO production caused by light treatment is measured. These measurements may be taken using a personal healthcare device comprising Fdots (e.g., a mouthpiece or a bandage) that is disposed adjacent to the tissue being evaluated.

At 856, the trained (baseline) ML model from ML algorithm 810 is applied to the data gathered at 854 and is used to predict the health status of the tissue at the particular time and location of the tissue where NO data are gathered. Notably, in certain representative embodiments, the determination of the health status of the tissue is the sole objective of execution of the method. In this case, the results of 856 are provided to the model, and at 842 an updating of the ML model is carried out. The updating of the ML model at 842 comprises updating of the weights of the model for the individual, and may include sharing of weights and parameters based on received federated learning rates, as described more fully in connection with Fig. 10.

As described more fully below, in accordance with a representative embodiment, a health map of the tissue of the individual can be made and updated at 856. In accordance with a representative embodiment, the ML algorithm 812 is applied iteratively at the particular location at other times. Based on the predicted health status of the tissue at the particular location, the health map can be generated. As described more fully below, an area in the health map that is related to the particular location is used to update the weights of the model at the particular location for the individual.

At 858, the trained (baseline) model from ML algorithm 812 is applied to the data gathered at 854 and to current and historical health status of the individual. The ML algorithm 812 then predict tissue treatment settings and thereby the recommended treatment. The resultant NO tissue response of the predicted treatment settings/treatment are also predicted at 858.

In this case, the results of 858 are provided to the model, and at 844 an updating of the ML model is carried out. The updating of the ML model at 842 comprises updating of the weights of the model for the individual, and may include sharing of weights and parameters based on received federated learning rates, as described more fully in connection with Fig. 10.

At 830, updating of the ML algorithm 812 is carried out. The predicted treatment settings predicted are then compared to a safety limit. As described more fully below, when the comparing of the predicted treatment settings is equal to or below the safety limit, the updated predicted treatment settings are stored and a tissue treatment at the predicted treatment settings can be carried out. However, when the comparison of the predicted settings is greater than the safety limit, treatment settings are adjusted. Weights of the trained computational model are then updated by retraining. Based on this re-training, treatment settings and predicted NO response are updated, and a comparison is made of the predicted treatment settings. This process continues until the predicted treatment settings are equal to or below the safety limit.

At 862, the treatment at the predicted treatment settings is carried out for the individual.

Fig. 9A is a flow-diagram 900 of baseline training of the computational model for predicting a health status of tissue in accordance with a representative embodiment. Certain aspects and details of the representative embodiments described above in connection with representative embodiments may be common to the description of the flow-diagram 900. These common aspects and details may not be repeated to avoid obscuring the presently described representative embodiments.

Real user data 901 comprise one or more of natural NO data 904, tissue health data 906, tissue temperature data 908, personal data 910, and other above-noted relevant data 912 for an individual and a large pool of other individuals. Notably with the exception of the personal data, these data are time-series data, which are a sequence of data points collected over time at consistent intervals, allowing analysis of how a variable changes over a specific period, often revealing patterns, trends, and seasonal fluctuations within the data. As will be appreciated, by real data is meant actual measured data and may comprise ground truth data useful in training the ML algorithm of various representative embodiments. Notably, in the event that a sufficient amount of real data are available, the model can be tested without the augmentation of synthetic data.

Synthetic data 902 comprise data that are generated using a simulation model. By way of illustration and not limitation, the simulation model may comprise a known a skin model or a known gums model that incorporates NO response to treatment for example. The simulation model may be based on study results of how skin or gums react to the treatment and why. By knowing the variables influencing this response it is possible to generate large amounts of data for different and very specific cases. It is also possible to build a simulation model and, based on the model, generate large amounts of data. As such, simulation models are well suited for training the ML model of various representative embodiments.

Synthetic data 902 comprise one or more of natural NO data 914, tissue health data 916, tissue temperature data 918, personal data 920, and other above-noted relevant data 922 for an individual and a large pool of other individuals. Notably with the exception of the personal data, these data are time-series data. As noted above, synthetic data are generated using a simulation model, and are used to supplement the real data.

At 924 the real and synthetic data are split into a training data set and a test data set, with the real and synthetic data being combined to provide the training data set and test data set. Notably, the data are split into the training data set and the test data set, where the training data set is used to train the ML algorithm, and the test data set is used for testing the ML algorithm. As will be appreciated, these data sets cannot overlap to avoid influencing the training of the ML algorithm. The synthetic data and the real data are combined to provide a sufficient test data and a sufficient training data set.

At 926 the training data set comprising real and synthetic data is realized, and at 928, the test data set comprising real and synthetic data is realized. At 930 and 932, respectively, the training data set and the test data set are normalized to fit training and test data to the model inputs by a known method.

The training data set is again split to provide input data 934 and output data 936. The input data 934 are used to train the ML algorithm used to predict the health status of tissue. The input data include natural NO data, temperature data, personal data and any other relevant data from the real and synthetic data 901, 902. The output data 936 include the predicted health of the tissue. These output data are tested against an output label using an error calculation the weights are tuned and provide the baseline weights of the LSTM model.

Similarly, at the test data set is again split to provide input data 937 and output data 938. The input data 937 are used to test the ML algorithm used to predict the health status of tissue. The input data 937 again include natural NO data, temperature data, personal data and any other relevant data from the real and synthetic data 901, 902. The output data 938 include the predicted health of the tissue. These output data are tested against an output label using an error calculation the weights are tuned and provide the baseline weights of the LSTM model.

At 940 in an example using an LSTM model, the labeled input data are used for training, and hyperparameters can be tuned during training to optimize the resulting predictions.. As noted above, in certain representative embodiments, the LSTM model performs better than other known ML models because it takes more history into account and it has a loop function. Notably, as the name implies, the LSTM model has a long term memory, or memory from a comparatively large number of NO measurements. Moreover, because the data are often time series data, the short term memory of the LSTM is beneficial. The result is a better estimate/prediction of the health of the tissue than can be realized using other known models. Moreover, at 940, training and test data are applied to the LSTM model according to the illustrative embodiment. Specifically, a training set of input and output pairs is provided to the LSTM model while training, and a set of input and output pairs of data is provided to the model to test the accuracy of the training of the LSTM model. Specifically, during training of the ML model transforming of input data and to output data belonging to that input data occurs to learn which outputs belong to particular inputs. Notably, test data are used to beneficially prevent overfitting of the model. In this way, the model will only use the training data during training. Next, the LSTM model will be tested against a separated data set the algorithm of the ML model did not train on to verify its performance.

As shown, this training will loop based on a loss function calculated during training beneficially to improve accuracy of the trained model by updating weights. In this way at 942, hyperparameters 942 can be tuned to optimize the training process on speed, learning rate and final results. The objective of this tuning during the training of the model is to provide faster and/or more accurate training results.

As alluded to above, one beneficial aspect of the various representative embodiments is the updating of the ML algorithm in a number of ways. As shown at 944 another input comprises updates to the weights of the LSTM model based on iterations of the training of the model for a particular individual and from applications of the model to other individuals. Aspects of these updates are described more fully in connection with federated learning in Fig. 10. As described more fully below, updates are realized by application of the LSTM model to an individual as ongoing treatment of the individual is carried out. Just by way of illustration, an individual may have a chronic problem with gingivitis, and as a result, NO emission measurements of the individual are made continually over a period of time. The iterative learning/re-training of the LSTM model enables finer tuning of the weights of the LSTM model with the goal of improving the assessment of the health of the tissue, and as described more fully in connection with Fig. 9B, improving the treatment by improving the prediction of the treatment settings and NO response. Moreover, updates are also used to improve the overall reliability of the LSTM model using federated learning as described below.

Fig. 9B is a flow-diagram 950 of baseline training of the computational model for predicting a tissue treatment settings and (NO) response of tissue in accordance with a representative embodiment. Certain aspects and details of the described above in connection with representative embodiments of Fig. 9B may be common to the description of the flow-diagram 900 and other representative embodiments described above. These common aspects and details may not be repeated to avoid obscuring the presently described representative embodiments.

Real user data 951 comprise one or more of natural NO data predicted health 954, treatment response natural and/or stimulated NO data 956 , tissue health data 958, tissue temperature data 960, personal data 962, other relevant data 964 as described above, and treatment details 965 for an individual and a large pool of other individuals. Notably, with the exception of the static personal data, these data are time-series data, which are a sequence of data points collected over time at consistent intervals, allowing analysis of how a variable changes over a specific period, often revealing patterns, trends, and seasonal fluctuations within the data. As will be appreciated, by real data is meant actual measured data and may comprise ground truth data useful in training the ML algorithm of various representative embodiments. Notably, in the event that a sufficient amount of real data is available, the model can be tested without the augmentation of synthetic data.

Synthetic data 952 comprise data that are generated using a simulation model such as described above. As noted above, the simulation model is within the purview of one of ordinary skill in the art.

Synthetic data 952 comprise artificial data generated from the simulation model's response of natural NO, tissue health data 964, treatment response NO data 966, tissue health data 968, tissue temperature data 972, personal data 971, other above-noted relevant data 974 and treatment details 975 for an individual model state and or variant and a large pool of other model variants. Notably with the exception of the static personal data, these data are time-series data.

At 974 the real and synthetic data are split into a training data set and a test data set, with the real and synthetic data being combined to provide the training data set and test data set. Notably, the data are split into the training data set and the test data set, where the training data set is used to train the ML algorithm, and the test data set is used for testing the ML algorithm. As will be appreciated, these data sets cannot overlap to avoid influencing the training of the ML algorithm. The synthetic data and the real data are combined to provide a sufficient test data and a sufficient training data set. Moreover, it is expected the models used to generate the synthetic data are not perfect and thus real user data is needed to cover these corner cases.

At 976 the training data set comprising real and synthetic data is realized, and at 978, the test data set comprising real and synthetic data is realized. At 980 and 982, respectively, the training data set and the test data set are normalized by a known method.

The training data set is again split to provide input data 984 and output data 986. The input data 984 are used to train the ML algorithm used to predict the health status of tissue. The input data include NO data, tissue health data, temperature data, personal data and any other relevant data from the real and synthetic data 951, 952. The output data 986 include the health of the tissue and/or the treatment settings and resultant NO response at the settings. These output data are used as target and or information for the algorithm and tested against, and using an error calculation to update the weights of the LSTM model. The input data belong to the output data with specific treatment settings and health status. This is used by the algorithm to learn what settings will cause the desired output in specific situations and specific health status.

Similarly, at the test data set is again split to provide input data 987 and output data 988. The input data 987 are used to test the ML algorithm used to predict the health status of tissue. The input data 987 again include NO data, tissue health data, temperature data, personal data and any other relevant data from the real and synthetic data 951, 952. The output data 988 include the treatment settings and resultant NO response at the settings. These output data are tested against an output of the algorithm during these training cycles based on the input. An error calculation is then used to update the weights of the LSTM model.

At 990, in accordance with a representative embodiment, an LSTM model is used for the ML model. As noted above, in certain representative embodiments, the LSTM model performs better than other known ML models because it takes more history into account and it has a loop function. Notably, as the name implies, the LSTM model has a long term memory, or memory from a comparatively large number of NO measurements. Moreover, because the data are often time series data, the short term memory of the LSTM is beneficial. The result is a better estimate/prediction of the health of the tissue than can be realized using other known models.

Applying the training and test data to the LSTM model, training the model begins with the model using test data to verify the training is improving the reliability of the model. To this end, and as noted above in the description of Fig. 9A, the training data set is used during training and the test data set is used to verify the improvement in the results from the LSTM model at 990.

As noted above, an error calculation is carried out to determine the reliability of the ML model being trained. When the current training step does not provide an acceptable result, the error value is used to update the weights of the LSTM model and at 990 with the adjustments to the weights 994, the LSTM is run again. This process continues until the error calculation is acceptable. Again, as described above with Fig. 9A, hyperparameters can be tuned to have faster or better training results. When training loops are completed the performance of the model is acceptable, the trained model is ready for deployment.

As alluded to above, one beneficial aspect of the various representative embodiments is the updating of the ML algorithm in a number of ways. As shown at 994 another input comprises updates to the weights of the LSTM model based on iterations of the model for a particular individual and from applications of the model to other individuals. Aspects of these updates are described more fully in connection with federated learning in Fig. 10. As described more fully below, updates are realized by application of the LSTM model to an individual as ongoing treatment of the individual is carried out. Just by way of illustration, an individual may have a chronic problem with gingivitis, and as a result, NO emission measurements of the individual are made continually over a period of time. The iterative learning/re-training of the LSTM model enables finer tuning of the weights of the LSTM model with the goal of improving the assessment of the health of the tissue and improving the treatment by improving the prediction of the treatment settings and NO response. Moreover, updates are also used to improve the overall reliability of the LSTM model using federated learning as described below.

Fig. 10 is a flow-diagram 1000 showing execution and updated training of a deployed computational model including personalization and federated learning based on a plurality of measurements of an individual and treatments of the individual in accordance with a representative embodiment. Certain aspects and details of the described above in connection with representative embodiments above may be common to the description of the flow-diagram 1000 and other representative embodiments described above.

Notably, the flow-diagram 1000 presents the execution of the baseline LSTM models trained for an individual in accordance with the representative embodiments described above in connection with Figs. 9A and 9B. In the interest of clarity, the flow-diagram 1000 includes both the LSTM model adapted to predict the health status of tissue of the individual, and the LSTM model adapted to predict the treatments settings and the NO response resulting from a treatment at the predicted treatment settings. One or both of these models may be executed by following the flow-diagram.

At 1002 personal history data and other relevant sensor data are collected. These other relevant sensor data may comprise data from previous treatments of the particular individual and relevant data such as health records of the individual, and/or data from other devices being used.

At 1004 local, natural NO production is measured, and at 1006 using the trained computational model (e.g., the LSTM trained in accordance with the representative embodiments of Fig. 9A) to predict the health status at a particular location of the individual.

At 1008 a comparison of the real tissue health with the previously predicted health is made (e.g., in Fig. 9A). As such, a verification and training step is carried out to determine whether the current health, NO reading is a match with the previously predicted levels again when there is no match the same error mechanism can be used to update / re-train the model. When the match is unsatisfactory based on an error calculation, during training at 1011, the weights of the LSTM model are updated to improve the chances of a match to the previous predictions. As will be appreciated, the retraining/updating of the model involves making minor adjustments to the weights with each iteration. This process is repeated with each session of the individual, enabling verification and, as needed, updating of the weights of the LSTM. As such, by the present technique, the LSTM model is repeatedly updated and retrained to further improve the accuracy of the LSTM model at 1011. Beneficially, therefore, at 1011, the LSTM model is continually updated with every future measurement/prediction sequence by the individual.

If only the health status prediction is desired, the process is interrupted until another measurement/prediction session is desired. Notably, according to a representative embodiment, later measurements and predictions of the health status at the same location can be used to provide a health map. Moreover, this process can be repeated at multiple locations to provide a more comprehensive health map.

To provide the predicted treatment settings an NO response, at 1010, the baseline LSTM model trained according to the representative embodiments of Fig. 9B is executed using the input data from the individual, and may include both the measured natural NO data and the stimulated NO data, as well as other data of the individual described above.

At 1012 the predicted treatment settings are measured against a safety level or other performance treatment threshold to verify that the treatment settings are at or below a treatment threshold.

When the predicted treatments settings are not within the prescribed safety limit, at 1011 the weights LSTM model are updated with the aim of realizing predicted treatment settings are optimized and that they are within the safety threshold. As will be appreciated, the retraining/updating of the model involves making minor adjustments to the weights with each iteration. The applied settings based on the predicted settings are adjusted to below the safety level to prevent safety problems hazards. One or multiple training loops at this stage can be used to update the models performance independent of the used settings at that stage. The weights of the model are updated by retraining based on the unacceptable results that are above the safety limit. Moreover, this process is repeated with each session of the individual, enabling retraining of the model by updating of the weights of the LSTM as needed to maintain safe treatment settings. As such, by these iterative techniques, the LSTM model is repeatedly updated and retrained to further improve the accuracy of the LSTM model at 1011. Beneficially, therefore, at 1011, the LSTM model is continually updated with every future measurement/prediction sequence by the individual.

At 1013, the treatment settings are updated and a treatment is carried out.

At 1014 measurements of the NO emission and other relevant sensor measurements such as temperature are carried out.

At 1016, after measurements are taken, a comparison of the predicted NO response to the measured NO response of the tissue from 1014 is made. When the measured response is not as predicted, the weights are again updated at 1011.

At 1018 the determination is made whether the treatment is completed. When it is not, the process repeats beginning at 1016. As will be appreciated, with each iteration of 1016-1018, weights of the model are continually updated (and the model is iteratively retrained) at 1011 enabling not only improvements to the accuracy of the individual's LSTM model, but also to the LSTM model used to provide a baseline model for other individuals.

In order to further refine the LSTM model on which each individual baseline LSTM model is based, at 1020, weights from the LSTM at 1011 are shared with a federated learning server 1022. As such, and without disclosing confidential data of the individual providing the weights to the LSTM model at 1011, the federated learning processed weights (i.e., averaging) can be shared, with other individuals 1030, 1034, 1036 and 1038, and back to 1011. Specifically, the processed weights from the individual provided to the LSTM model at 1011 and from weights 1031, 1033, 1035 and 1037 from individuals 1030, 1034, 1036 and 1038, respectively, are shared via a federated learning server 1026, which is remote to the individuals, such as at the company that creates the methods and systems of the present teachings. Notably, the federated learning server may be in the cloud via a known cloud-based system.

The shared weights are provided from the federated learning server 1026 to a federated learning algorithm and as shown at 1028, these updated weights are shared with the individuals 1030, 1032, 1034, 1036 and 1038.

It is noted that the line between the shared weights at 1020 and the LSTM model at 1011 is a double-headed arrow signifying the sharing of weights back and forth between the individuals of the network. As such, weights of an individual's LSTM model are shared only with the federated learning server 1026, and weights from other individuals of the network (e.g. individuals 1030, 1032,1034, 1036 and 1038) are not shared with individuals as for example 1011 via the federated learning server 1026. Notably, however, updated and combined weights do not replace individual weights at 1011, but rather update these weights. Rather, the weights of an individual's LSTM algorithm are updated with federated learning algorithm weights; and the individual continues to receive weights via the updating described with the left side of the flow-diagram 1000 in Fig. 10.

Finally, it is noted that the sequence from 1002-1011 is also applied to individuals 1030, 1032, 1034 and 1036, thereby improving the speed and/or accuracy of the applied ML model.

In the last analysis, the weights are updated in comparatively small-magnitude steps that each have a small impact on the baseline LSTM model. Through this iterative process, the reliability and performance baseline LSTM model is improved.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. A system for predicting tissue treatment settings and response of tissue, the system comprising:
a personal healthcare device comprising fluorescent dots (Fdots) adapted to detect nitric oxide (NO);
a memory adapted to store a trained computational model comprising instructions; and
a processor, wherein the instructions, when executed by the processor, cause the processor to:
(a) receive measured nitric oxide (NO) tissue data from a personal healthcare device;
(b) apply a trained computational model to predict an NO response, and based on the predicted NO response, predict tissue treatment settings;
(c) compare the predicted treatment settings to a safety limit;
(d) when the comparison of the predicted treatment settings is equal to or below the safety limit, update the predicted treatment settings and carrying out the tissue treatment at the predicted treatment settings; and
(e) when the comparison of the prediction is greater than the safety limit, adjust the tissue treatment settings, update weights of the trained computational model, and repeat (c) through (e).

2. The system of claim 1, wherein, based on the measured NO tissue data, the instructions further cause the processor to predict a health status of the tissue.

3. The system of claim 2, wherein the instructions further cause the processor to estimate treatment settings based on the predicted health status of the tissue.

4. The system of claim 1, wherein after the tissue treatment settings are predicted, the instructions further cause the processor to predict an NO tissue response to a treatment at the predicted tissue treatment settings.

5. The system of claim 1, wherein after the carrying out of the tissue treatment, the instructions further cause the processor to measure NO production with the personal healthcare device and compare a tissue response to a predicted response of the tissue.

6. The system of claim 5, wherein when the comparing of the tissue response is not equal to the predicted response, the instructions cause the processor to repeat (c) through (e).

7. The system of claim 1, wherein the trained computational model is a deployed computational model, and when updating the weights of the trained computational model, the instructions further cause the processor to adjust parameters of the trained computational model.

8. The system of claim 1, wherein the trained computational model is a deployed computational model, and the instructions further cause the processor to:
adjust parameters of the trained computational model based on a real-time response of the tissue.

9. The system of claim 8, wherein the real-time response is based on a treatment of the tissue with a particular NO level.

10. The system of claim 1, wherein the instructions further cause the processor to apply the trained computational model to a particular location at a particular time; and repeat (c) through (e).

11. The system of claim 10, wherein the instructions further cause the processor to apply the trained computational model at the particular location at another particular time; repeating (c) through (e); and generate a health map.

12. The system of claim 11, wherein the instructions further cause the processor to use an area in the health map that is related to the particular location to update the weights of the model at the particular location.

13. The system of claim 1, wherein the trained computational model is a deployed computational model, and the instructions further cause the processor to:
share weights and parameters based on received federated learning rates.

14. A tangible, non-transitory computer readable medium that stores instructions, which when executed by a processor, cause the processor to:
(a) receive measured nitric oxide (NO) tissue data from a personal healthcare device;
(b) apply a trained computational model to the NO tissue data to predict an NO response, and based on the predicted NO response, predict tissue treatment settings;
(c) compare the predicted treatment settings to a safety limit;
(d) when the comparison of the predicted treatment settings is equal to or below the safety limit, update the predicted treatment settings and carrying out the tissue treatment at the predicted treatment settings; and
(e) when the comparison of the prediction is greater than the safety limit, adjust the tissue treatment settings, update weights of the trained computational model, and repeat (c) through (e).

15. A method of predicting tissue treatment settings and nitric oxide (NO) response of tissue, the method comprising:
(a) measuring nitric oxide (NO) tissue data from a personal healthcare device comprising fluorescent dots (Fdots);
(b) applying a trained computational model to the NO tissue data to predict an NO response, and based on the predicted NO response, predicting tissue treatment settings;
(c) comparing the predicted treatment settings to a safety limit;
(d) when the comparing of the predicted treatment settings is equal to or below the safety limit, updating the predicted treatment settings and carrying out tissue treatment at the predicted treatment settings; and
(e) when the comparing of the prediction is greater than the safety limit, adjusting the tissue treatment settings, updating weights of the trained computational model, and repeating (c) through (e).
